# EUROPÄISCHE PATENTANMELDUNG

(11) **EP 4 703 348 A1**
(43) Veröffentlichungstag der Anmeldung: **04.03.2026**
(21) Anmeldenummer: 24198041.6
(22) Anmeldetag: 03.09.2024
(51) Int. Cl.: C07C 69/608, C08K 5/00

(54) **MISCHESTER DER 1,2,4-CYCLOHEXANTRIPROPIONSÄURE UND EINE WEICHMACHERZUSAMMENSETZUNG ENTHALTEND DIE MISCHESTER**

(71) Anmelder: Evonik Oxeno GmbH & Co. KG, 45772 Marl (DE)
(72) Erfinder: GRASS, Michael, 45721 Haltern am See (DE); RUSEK, Monika, 45149 Essen (DE); VAN EICKELS, Michael, 45657 Recklinghausen (DE)
(74) Vertreter: Evonik Patent Association

(57) **Zusammenfassung**

Die vorliegende Erfindung betrifft eine Weichmacherzusammensetzung enthaltend eine Verbindung der Formel (1) 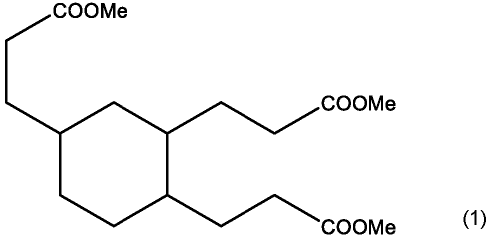 und mindestens einen Dialkylterephthalsäureester, wobei die beiden Alkylgruppen im Dialkylterephthalsäureester jeweils 8 oder 9 Kohlenstoffatome aufweisen.

## Beschreibung

Gegenstand der Erfindung ist eine Mischung von Triestern der 1,2,4-Cyclohexantripropionsäure der nachfolgenden Formel (1) 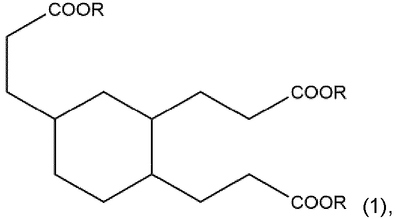 wobei 5 bis 75 Mol% der Reste R Methylreste und 25 bis 95 Mol% der Reste R ein C2- bis C1 0-Alkylrest sind.

Die erfindungsgemäßen Verbindungen nach der Formel (1) sind Mischester der 1,2,4-Cyclohexantripropionsäure. Trimethylester der 1 ,2,4-Cyclohexantripropionsäure, bei denen alle Reste R Methylreste sind, sind grundsätzlich bekannt und beispielsweise in der EP 3 838 886 A1 beschrieben worden. Dort wurde auch erwähnt, dass diese Trimethylester der 1,2,4-Cyclohexantripropionsäure als Weichmacher eingesetzt werden können. Der Trimethylester wurde in diesem Zusammenhang als ein Produkt mit niedriger Geliertemperatur beschrieben.

Die Trimethylester der 1,2,4-Cyclohexantripropionsäure zeigen beim Einsatz als Weichmacher in Kunststoffen nicht immer nur positive Eigenschaften. Würde man den Trimethylester als Weichmacher, beispielsweise in einer PVC-basierten Plastisol-Rezeptur einsetzen, würde das Plastisol eine recht hohe Viskosität aufweisen. Außerdem würde bei der Verarbeitung des Plastisols ein nicht unerheblicher Anteil des Trimethylesters verdampfen und somit nicht mehr im Endprodukt verbleiben.

Der vorliegenden Erfindung lag nun die Aufgabe zugrunde einen Mischester bereitzustellen, der bessere anwendungstechnische Eigenschaften aufweist der Trimethylester der 1,2,4-Cyclohexantripropionsäure.

Diese Aufgabe wird durch den Mischester nach Anspruch 1 gelöst. Gegenstand der Erfindung ist demnach eine Mischung von Triestern der 1,2,4-Cyclohexantripropionsäure gemäß der nachfolgenden Formel (1) 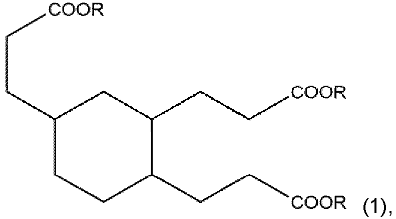 wobei in der Mischung 5 bis 75 Mol% der Reste R Methylreste und 25 bis 95 Mol% der Reste R ein C2-bis C10-Alkylrest sind. Bevorzugte Ausführungsformen der vorliegenden Erfindung sind in den Unteransprüchen angegeben. Die Prozentangabe (Mol%) bezieht sich jeweils auf alle Reste R in der Mischung.

In einer bevorzugten Ausführungsform der vorliegenden Erfindung liegt eine Mischung von Triestern der 1,2,4-Cyclohexantripropionsäure gemäß der nachfolgenden Formel (1) vor, 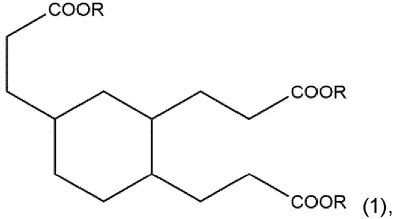 wobei in der Mischung 15 bis 70 Mol%, besonders bevorzugt 20 bis 65 Mol% der Reste R Methylreste und 30 bis 85 Mol%, besonders bevorzugt 35 bis 80 Mol% der Reste R C2- bis C10-Alkylreste sind. Die Prozentangabe Mol% bezieht sich auch hier jeweils auf 100 Mol% der Reste R, also alle Reste R in der Verbindung nach Formel (1) in der Mischung.

Der C2- bis C10-Alkylrest, der zu dem beanspruchten Anteil in den Triestern der 1,2,4-Cyclohexantripropionsäure gemäß Formel (1) der erfindungsgemäßen Mischung enthalten sind, besteht aus Ethylgruppen, Propylgruppen, Butylgruppen, Pentylgruppen, Hexylgruppen, Heptylgruppen, Ocylgruppen, Nonylgruppen oder Decylgruppen. Unter die genannten Alkylgruppen fallen sowohl alle Isomere der entsprechenden Alkylgruppe als auch Mischungen aus verschiedenen Isomeren der entsprechenden Alkylgruppe. Unter den Begriff "Pentylgruppen" fallen beispielsweise zumindest die 2-Methylbutylgruppe, die n-Pentylgruppe und die 3-Methylbutylgruppe. Zu den "Nonylgruppen" gehören beispielsweise n-Nonylgruppen oder Isononylgruppen. Unter einer Isononylgruppe wird im Sinne der vorliegenden Erfindung eine Mischung aus linearen und verzweigten C9-Alkylgruppen verstanden.

Der C2- bis C10-Alkylrest ist in einer bevorzugten Ausführungsform ein C4- bis C9-Alkylrest, besonderes bevorzugt ein C5- bis C9-Alkylrest. In einer bevorzugten Ausführungsform der vorliegenden Erfindung liegt somit eine Mischung von Triestern der 1,2,4-Cyclohexantripropionsäure gemäß der nachfolgenden Formel (1) vor, 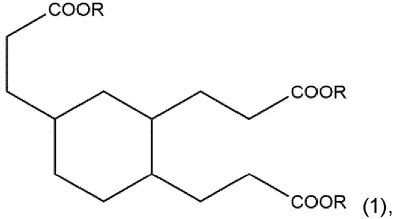 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C4- bis C9-Alkylrest, vorzugsweise ein C5- bis C9-Alkylrest sind. Die Prozentangabe (Mol%) bezieht sich auch hier jeweils auf alle Reste R in der Mischung.

Der C4- bis C9-Alkylrest bzw. der C5- bis C9-Alkylrest, der zu dem beanspruchten Anteil in den Triestern der 1,2,4-Cyclohexantripropionsäure gemäß Formel (1) der bevorzugten erfindungsgemäßen Mischung enthalten sind, besteht aus Butylgruppen, Pentylgruppen, Hexylgruppen, Heptylgruppen, Ocylgruppen, Nonylgruppen. Unter die genannten Alkylgruppen fallen sowohl alle Isomere der entsprechenden Alkylgruppe als auch Mischung aus verschiedenen Isomeren der entsprechenden Alkylgruppe.

In einer besonders bevorzugten Ausführungsform der vorliegenden Erfindung ist der C2- bis C10-Alkylrest eine Pentylgruppe oder eine Isononylgruppe. Unter den Begriff "Pentylgruppen" fallen beispielsweise zumindest die 2-Methylbutylgruppe, die n-Pentylgruppe und die 3-Methylbutylgruppe oder Mischungen davon. Unter einer Isononylgruppe wird im Sinne der vorliegenden Erfindung eine Mischung aus linearen und verzweigten C9-Alkylgruppen verstanden.

In einer besonders bevorzugten Ausführungsform der vorliegenden Erfindung liegt somit eine Mischung von Triestern der 1,2,4-Cyclohexantripropionsäure gemäß der nachfolgenden Formel (1) vor, 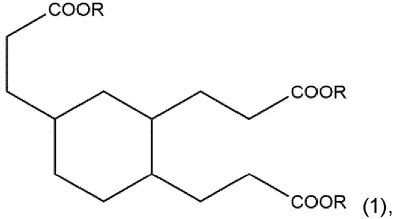 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R eine Pentylgruppe oder eine Isononylgruppe sind. Die Prozentangabe (Mol%) bezieht sich auch hier jeweils auf alle Reste R in der Mischung.

Der Mischung von Triestern der 1,2,4-Cyclohexantripropionsäure ist aktuell nicht kommerziell erhältlich. Die Herstellung kann aber über die Umesterung des Trimethylesters der 1,2,4-Cyclohexantripropionsäure mit einer entsprechenden unterstöchiometrischen Menge an einem C2- bis C10-Alkohol, vorzugsweise einem C4- bis C9-Alkohol, besonders bevorzugt einem C5- bis C9-Alkohol und ganz besonders bevorzugt Pentanol oder Isononanol erfolgen Ein Verfahren zur Herstellung der Trimethylester der 1,2,4-Cyclohexantripropionsäure wird beispielsweise in der EP 3 842 411 A1 beschrieben. Die Ester der 1,2,4-Cyclohexantripropionsäure sind somit allgemein zugänglich.

Gegenstand der vorliegenden Erfindung ist auch eine Weichmacherzusammensetzung, die eine Mischung von Triestern der 1,2,4-Cyclohexantripropionsäure gemäß der nachfolgenden Formel (1) 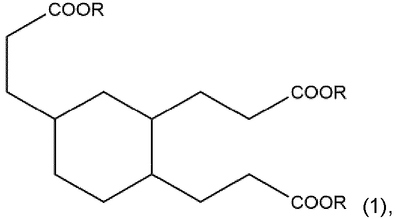 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, und mindestens einen weiteren Weichmacher enthält. Die oben genannten Prozentangaben (Mol%) beziehen sich auch hier jeweils auf alle Reste R in der Mischung.

Die beiden Substanzen, d. h. der Mischester und der weitere Weichmacher, können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine veränderte weichmachende Wirkung eintritt. In einer bevorzugten Ausführungsform der vorliegenden Erfindung liegt die erfindungsgemäße Mischung von Triestern der 1,2,4-Cyclohexantripropionsäure gemäß der nachfolgenden Formel (1) und der weitere Weichmacher in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vor.

Die erfindungsgemäße Weichmacherzusammensetzung kann darüber hinaus noch mindestens einen weiteren Weichmacher enthalten. Der weitere Weichmacher wird vorzugsweise aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Cyclohexandicarboxylaten, Trimellitaten, Phthalaten, Triestern der 1,2,4-Trialkylpropionsäure mit Ausnahme der bereits vorhandenen Verbindungen und oligomeren oder polymeren Estern auf Basis von Adipin-, Bernstein- oder Sebacinsäure, ausgewählt.

In einer weiterhin bevorzugten Ausführungsform wird der mindestens eine weitere Weichmacher aus der Gruppe, bestehend aus Alkylbenzoaten, Alkylsulfonsäureestern des Phenols, Dialkyladipaten, Glycerinestern, C4- bis C6- Alkansäureester von Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, Dialkylestern der 1,2-, 1,3- oder 1,4-Cyclohexandicarbonsäure, ausgewählt.

In einer besonders bevorzugten Ausführungsform wird der mindestens eine weitere Weichmacher, der in der erfindungsgemäßen Weichmacherzusammensetzung enthalten ist, aus der Gruppe, bestehend aus C8- bis C13-Alkylbenzoaten, C4- bis C10-Dialkyladipaten, Pentaerythrittetravalerat, acetylierten oder nicht acetylierten Citronensäuretrialkylestern mit C4 bis C9-Alkylgruppen, C4- bis C10-Trialkyltrimellitaten, C4- bis C9-Dialkylterephthalaten, C4- bis C13-Dialkylphthalaten, insbesondere C9-bis C13-Dialkylphthalaten und C4- bis C10-Dialkylestern der 1,2-, 1,3 oder 1,4-Cyclohexandicarbonsäure, ausgewählt.

Ein Gegenstand der vorliegenden Erfindung ist demnach eine Weichmacherzusammensetzung, die den Tributylcitrat und eine Mischung von Triestern der 1,2,4-Cyclohexantripropionsäure gemäß der nachfolgenden Formel (1) 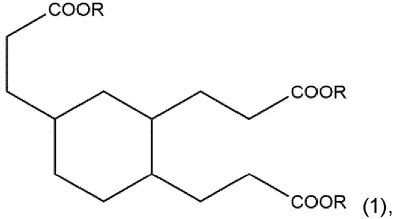 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Tributylcitrat liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Tripentylcitrat und eine Mischung von Triestern der 1,2,4-Cyclohexantripropionsäure gemäß der nachfolgenden Formel (1) 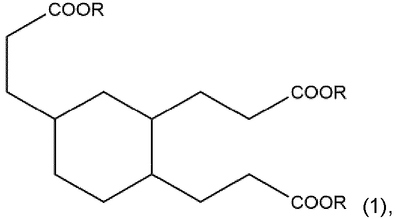 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Tripentylcitrat liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Acetyltributylcitrat (ATBC) und eine Mischung von Triestern der 1,2,4-Cyclohexantripropionsäure gemäß der nachfolgenden Formel (1) 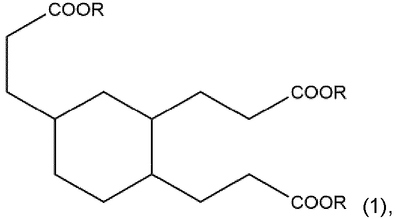 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Acetyltributylcitrat (ATBC) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Acetyltripentylcitrat und eine Mischung von Triestern der 1,2,4-Cyclohexantripropionsäure gemäß der nachfolgenden Formel (1) 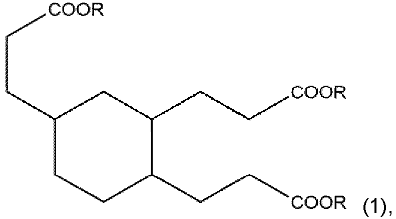 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Acetyltripentylcitrat liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Diethylhexyladipat (DEHA) und eine Mischung von Triestern der 1,2,4-Cyclohexantripropionsäure gemäß der nachfolgenden Formel (1) 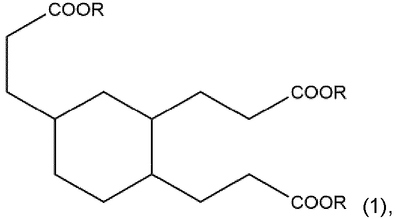 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Diethylhexyladipat (DEHA) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Diisononyladipat (DINA) und eine Mischung von Triestern der 1,2,4-Cyclohexantripropionsäure gemäß der nachfolgenden Formel (1) 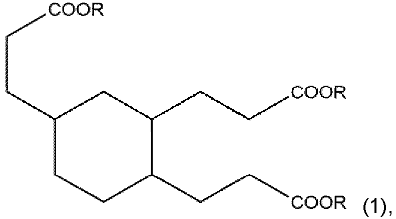 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Diisononyladipat (DINA) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Dibutylterephthalat (DBT) und eine Mischung von Triestern der 1,2,4-Cyclohexantripropionsäure gemäß der nachfolgenden Formel (1) 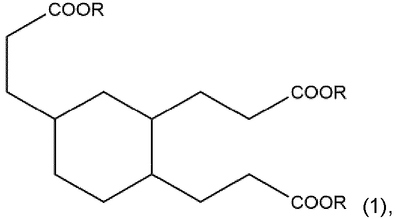 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Dibutylterephthalat (DBT) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Dipentylterephthalat (DPT) und eine Mischung von Triestern der 1,2,4-Cyclohexantripropionsäure gemäß der nachfolgenden Formel (1) 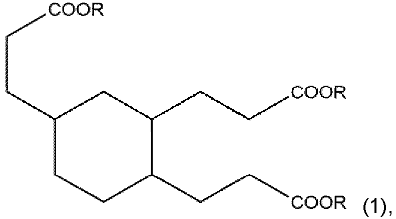 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Dipentylterephthalat (DPT) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Di-2-ethylhexylterephthalat (DOTP) und eine Mischung von Triestern der 1,2,4-Cyclohexantripropionsäure gemäß der nachfolgenden Formel (1) 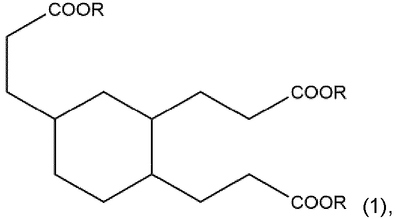 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Di-2-ethylhexylterephthalat (DOTP) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den Diisononylterephthalat (DINT) und eine Mischung von Triestern der 1,2,4-Cyclohexantripropionsäure gemäß der nachfolgenden Formel (1) 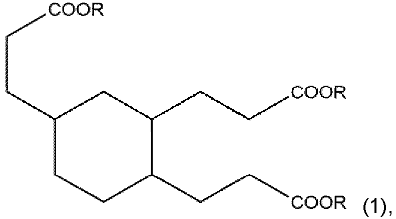 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und Diisononylterephthalat (DINT) liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den 1,2-Diisononylcyclohexandicarbonsäureester (DINCH) und eine Mischung von Triestern der 1,2,4-Cyclohexantripropionsäure gemäß der nachfolgenden Formel (1) 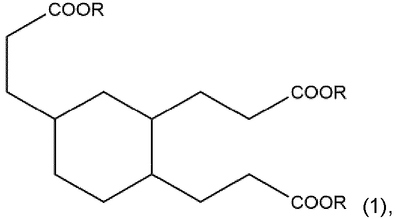 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und DINCH liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den 1,4-Diethylhexylcyclohexandicarbonsäureester (1,4-DEHCH) und eine Mischung von Triestern der 1,2,4-Cyclohexantripropionsäure gemäß der nachfolgenden Formel (1) 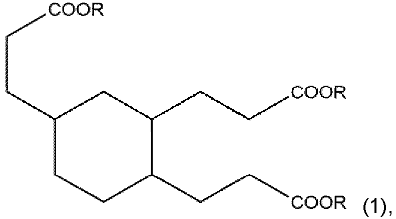 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und 1,4-DEHCH liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den 1,4-Diisononylcyclohexandicarbonsäureester (DINCD) und eine Mischung von Triestern der 1,2,4-Cyclohexantripropionsäure gemäß der nachfolgenden Formel (1) 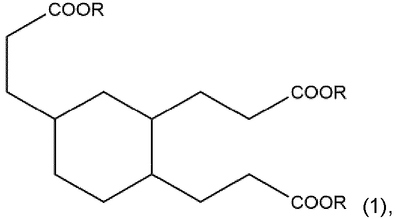 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und DINCD liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die den 1,2-Di-2-ethylhexylcyclohexandicarbonsäureester (1,2-DEHCH) und eine Mischung von Triestern der 1,2,4-Cyclohexantripropionsäure gemäß der nachfolgenden Formel (1) 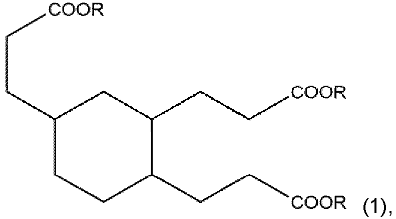 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und 1,2-DEHCH liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die Triisononyltrimellitat (TINTM) und eine Mischung von Triestern der 1,2,4-Cyclohexantripropionsäure gemäß der nachfolgenden Formel (1) 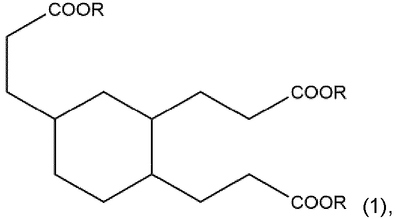 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und TINTM liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die Tripentyltrimellitat (TPTM) und eine Mischung von Triestern der 1,2,4-Cyclohexantripropionsäure gemäß der nachfolgenden Formel (1) 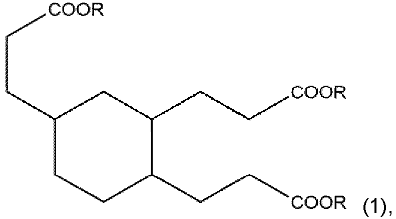 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und TPTM liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Ein Gegenstand der vorliegenden Erfindung ist demnach eine Weichmacherzusammensetzung, die Triethylhexyltrimellitat (TEHTM bzw. TOTM) und eine Mischung von Triestern der 1,2,4-Cyclohexantripropionsäure gemäß der nachfolgenden Formel (1) 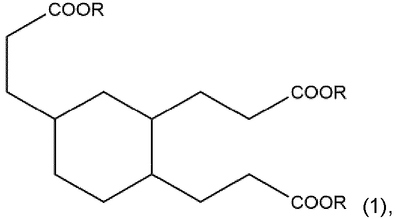 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, enthält. Die erfindungsgemäße Mischung von Triestern der 1,2,4-Cyclohexantripropionsäure und TOTM liegen in einem Gewichtsverhältnis (Mischung von Triestern : weiterer Weichmacher) von 1 : 99 bis 99 : 1, vorzugsweise 10 : 90 bis 90 : 10, besonders bevorzugt 30 : 70 bis 70 : 30 oder 50 : 50 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o. g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Mischung von Triestern der 1,2,4-Cyclohexantripropionsäure oder der Gesamtsumme aus der Mischung von Triestern der 1,2,4-Cyclohexantripropionsäure und dem weiteren Weichmacher eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Mischung von Triestern der 1 ,2,4-Cyclohexantripropionsäure oder der Gesamtsumme aus der Mischung von Triestern der 1,2,4-Cyclohexantripropionsäure und dem weiteren Weichmacher in der Weichmacherzusammensetzung enthalten sein.

Ein weiterer Gegenstand der vorliegenden Erfindung ist eine Kunststoffzusammensetzung, die einen Kunststoff und Mischung von Triestern der 1,2,4-Cyclohexantripropionsäure oder die Weichmacherzusammensetzung, umfassend die Mischung von Triestern der 1,2,4-Cyclohexantripropionsäure und mindestens einen weiteren Weichmacher enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1 - Herstellung von Cyclohexan-1,2,4-tripropionsäuretrimethylester (Me-Tc)

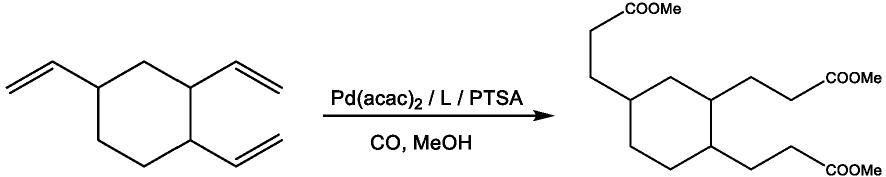

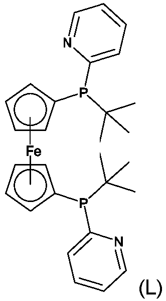

[Pd(acac)2] (15,2 mg, 0,1 mol%), der Katalysator L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

### Beispiele 2a und 2b - Herstellung von Mischestern der 1,2,4-Cyclohexantricarbonsäure mit Methanol und Pentanol

Der nach Beispiel 1 hergestellte Trimethylester (Me-Tc) wurde in einer typischen Umesterungsapparatur im Labor mit n-Pentanol zum Produkt MPe-Tc umgeestert. Die Umesterungsapparatur besteht aus einem Glaskolben, der mit einem Rührer, Temperaturfühler, einer Destillationskolonne, Vakuumteiler und Destillationsaufbau ausgestattet ist.

Zunächst wurden für Beispiel 2a 150 g n-Pentanol und 484 g M-Tc in der Umesterungsapparatur vorgelegt. Im Anschluss wurde die gesamte Apparatur mit Stickstoff gespült. und danach 0,89 g Tetra(n-butyl)titanat (TNBT) als Katalysator hinzugegeben. Danach wurde die Reaktion durch Aufheizen gestartet. Die Reaktionstemperatur betrug dabei 130 - 240°C, d. h. sie stieg während der Reaktion an. Methanol, dass bei der Umesterung entstand, wurde über die Kolonne und Destillationsapparatur aus dem System entfernt (65°C Kopftemperatur). Der C5-Alkohol, welche in der Kolonne vom Methanol getrennt wurde, wurde als Rückfluss wieder in das Reaktionsgemisch zurückgeführt. Als kein Destillatanfall mehr zu beobachten war, wurde die Reaktion beendet

Im Anschluss an die Reaktion, wurde der überschüssige Alkohol bei 160 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 160 °C mit Stickstoff für 2 h gestrippt. Im letzten Schritt wird das Produkt über einen 0,5 µm PTFE-Filter und Perlite als Filterhilfsmittel klar filtriert.

Die Synthese für Beispiel 2 b wurde analog durchgeführt mit 222 g n-Pentanol, 378 g M-Tc und 0,68 g TNBT.

Der genaue Anteil von Methyl- und Pentylgruppen in Mol-% wurde im Anschluss der Reaktionen mittels 1H-NMR bestimmt, die Anteile der unterschiedlichen Ester mittels GC in Flächen-%

**Tabelle 1: Eduktverhältnisse und Zusammensetzungen der Triester**

| | Äquivalente n-Pentanol pro Mol Me-Tc in der Synthese | Anteil Methylester im Produkt | Anteil Pentylester im Produkt | |
|---|---|---|---|---|
| 2a* | 1,2 | 59 | 41 | |
| 2b* | 2,3 | 25 | 75 | |
| | | | | |

| | FI. -% Trimethylester | FI.-% Dimethylmonopentylester | FI.-% Monomethyldipentylester | FI.-% Tripentylester |
|---|---|---|---|---|
| 2a* | 15,6 | 41,2 | 34,2 | 8,8 |
| 2b* | 1,1 | 11,6 | 40,8 | 46,4 |

| | | | | |
|---|---|---|---|---|
| * erfindungsgemäß | | | | |

### Beispiele 3a und 3b - Herstellung von Mischestern der 1,2,4-Cyclohexantricarbonsäure mit Methanol und Isononanol

Der nach Beispiel 1 hergestellte Trimethylester (Me-Tc) wurde in einer typischen Labor Umesterungsapparatur wie in Beispiel 2a /2b mit Isononanol zum Produkt In-Tc umgeestert.

Zunächst wurden für Beispiel 3a 190 g Isononanol (INA, Hersteller Evonik Oxeno GmbH & Co. KG) für Beispiel 3a und 376 g M-Tc in der Umesterungsapparatur vorgelegt. Im Anschluss wurde die gesamte Apparatur mit Stickstoff gespült. Als nächstes wurden 0,37 g Tetra(n-butyl)titanat (TNBT) als Katalysator hinzugegeben. Danach wurde die Reaktion durch Aufheizen gestartet. Die Reaktionstemperatur betrug dabei 130 - 240°C., d. h. sie stieg während der Reaktion an. Methanol, das bei der Umesterung entstand, wurde über die Kolonne und Destillationsapparatur aus dem System entfernt (65°C Kopftemperatur). INA, dass in der Kolonne vom Methanol getrennt wurde, wurde als Rückfluss wieder in das Reaktionsgemisch zurückgeführt. Gelegentlich wurde leichtes Vakuum angelegt, um ausreichend Destillat zu erhalten. Als kein Destillatanfall mehr zu beobachten war wurde die Reaktion beendet.

Im Anschluss an die Reaktion, wurde der überschüssige Alkohol bei 180 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Als nächstes wurde die Mischung bei 180 °C mit Stickstoff für 2 h gestrippt. Im letzten Schritt wird das Produkt über einen 0,5 µm PTFE-Filter und Perlite als Filterhilfsmittel klar filtriert.

Die Synthese für Beispiel 3b wurde analog durchgeführt mit 288 g Isononanol, 301 g M-Tc und 0,45 g TNBT.

Der genaue Anteil von Methyl- und Isononylgruppen in Mol-% wurde im Anschluss der Reaktionen mittels 1H-NMR bestimmt, die Anteile der unterschiedlichen Ester mittels GC in Flächen-%

**Tabelle 2: Eduktverhältnisse und Zusammensetzungen der Triester**

| | Äquivalente n-Isononanol pro Mol Me-Tc in der Synthese | Anteil Methylester im Produkt | Anteil Pentylester im Produkt | |
|---|---|---|---|---|
| 3a* | 1,2 | 60 | 40 | |
| 3b* | 2,3 | 23 | 77 | |
| | | | | |

| | FI. -% Trimethylester | FI.-% Dimethylmonoisononylester | FI.-% Monomethyldiisononylester | FI.-% Triisononylester |
|---|---|---|---|---|
| 3a* | 12,8 | 42,1 | 36,3 | 8,2 |
| 3b* | 0,5 | 9,4 | 42,4 | 46,8 |

| | | | | |
|---|---|---|---|---|
| * erfindungsgemäß | | | | |

### Beispiel 4 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezeptur der Polymerzusammensetzung ist in den Tabellen 3 und 4 aufgelistet.

**Tabelle 3: Übersicht der hergestellten Plastisole (Teil 1)**

| Plastisol | 1 | 2* | 3* |
|---|---|---|---|
| | phr | phr | phr |
| PVC (Vestolit^{®} P 1430 K 70 Ultra; Fa. Vestolit) | 100 | 100 | 100 |
| Me-Tc | 50 | | |
| C1/C5- Mischester 59/41 (Bsp. 2a) | | 50 | |
| C1/C5-Mischester 25/75 (Bsp. 2b) | | | 50 |
| epoxidiertes Sojabohnenöl als Co-Stabilisator (Edenol^{®} D81, Fa. Emery) | 3 | 3 | 3 |
| Thermostabilisator auf Ca/Zn-Basis (Reagens MBL 197/9 PF) | 2 | 2 | 2 |

| | | | |
|---|---|---|---|
| * = erfindungsgemäße Zusammensetzung phr = parts per hundred parts resin | | | |

**Tabelle 4: Übersicht der hergestellten Plastisole (Teil 2)**

| Plastisol | 4* | 5* |
|---|---|---|
| | phr | phr |
| PVC (Vestolit^{®} P 1430 K 70 Ultra; Fa. Vestolit) | 100 | 100 |
| C1/C9-Mischester 60:40 (Bsp. 3a) | 50 | |
| C1/C9-Mischester 23:77 (Bsp. 3b) | | 50 |
| epoxidiertes Sojabohnenöl als Co-Stabilisator (Edenol^{®} D81, Fa. Emery) | 3 | 3 |
| Thermostabilisator auf Ca/Zn-Basis (Reagens MBL 197/9 PF) | 2 | 2 |

| | | |
|---|---|---|
| * = erfindungsgemäße Zusammensetzung phr = parts per hundred parts resin | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 5 - Messung der Viskositäten der Plastisole

Die Messung der Viskosität der in Beispiel 4 hergestellten Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden.

Während der Messung wurden folgende Punkte angesteuert:
- Vorscherung von 100 s-1 für einen Zeitraum von 60 Sekunden, bei der keine Messwerte aufgenommen wurden
- Scherraten-Abwärtsrampe von 200 s-1 bis 0,1 s-1. Es wurden 30 Messpunkte aufgenommen mit einer Messpunktdauer von je 10 Sekunden.

Die Messungen wurden bei Raumtemperatur durchgeführt. Die Ergebnisse der Viskositätsmessungen der in Beispiel 4 hergestellten Plastisole mit den jeweils angegebenen Weichmachern bzw.

Weichmachermischungen sind in Tabelle 5 gezeigt (Messung 2h und 24 h bei einer Schergeschwindigkeit von100 s⁻¹.

**Tabelle 5: Ergebnisse der Viskositätsmessung**

| Rezeptur | Viskosität bei 100 s⁻¹ nach 2h / Pa*s | Viskosität bei 100 s⁻¹ nach 24h / Pa*s | Anstieg von 2h nach 24 h /% |
|---|---|---|---|
| 1 | 14,9 | 19,5 | 31,4 |
| 2* | 7,1 | 9,2 | 28,5 |
| 3* | 5,2 | 6,2 | 18,2 |
| 4* | 10,7 | 12,0 | 12,7 |
| 5* | 13,5 | 12,3 | - 8,8 |

| | | | |
|---|---|---|---|
| * = erfindungsgemäß | | | |

Gegenüber dem Trimethylester weisen die erfindungsgemäßen Mischester 2* bis 5* sowohl ein niedrigeres Viskositätsniveau auf als auch eine geringere Viskositätsänderung (Prozentuale Änderung von 2h auf 24 h), womit eine bessere Verarbeitbarkeit des Plastisols ermöglicht wird.

### Beispiel 6 - Herstellung von Folien

Die im Beispiel 4 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 7 - Bestimmung des Masseverlusts (Aktivkohle-Verfahren)

In Anlehnung an DIN EN ISO 176 (Verfahren B) wurden aus den hergestellten PVC- Folien jeweils drei kreisrunde Scheiben mit einem Durchmesser von 5 cm ausgestanzt und zunächst für 16 Stunden im Exsikkator bei Raumtemperatur konditioniert. Auf der Analysenwaage wurden die Proben-Kreise ausgewogen, in einen Drahtkorb gelegt, welcher mit einer Klammer verschlossen wurde. Die Drahtkörbe wurden anschließend in eine mit Aktivkohle gefüllte Weißblechdose so platziert, dass sich zwischen den einzelnen Körben bzw. Korb und Deckel (gelocht) bzw. Boden jeweils etwa 130 ml Aktivkohle befanden.

Die befüllten Weißblechdosen wurden im vortemperierten Heizschrank (120 °C) so platziert, dass der Lüfter im Schrank nicht zugestellt wurde und die Dosen sich nicht berührten. Luftwechsel und Abluftklappe wurden auf jeweils 10 % eingestellt. Nach 72 Stunden wurden die Dosen wieder aus dem Schrank entnommen, abgekühlt, danach die einzelnen Proben den Körben entnommen, im Exsikkator nochmals 16 Stunden konditioniert und danach auf der Analysenwaage zurückgewogen. Die erhaltene Massedifferenz entsteht durch den Verlust an Weichmacher. Von den jeweils drei Massedifferenzen wurde der Mittelwert gebildet und prozentual der Verlust an Weichmacher errechnet. In Tabelle 6 sind die Ergebnisse der Flüchtigkeiten aufgeführt.

**Tabelle 6: Massenverlust der Prüfkörper**

| Foliennummer | Massenverlust nach 3 Tagen in % |
|---|---|
| 1 | 7,3 |
| 2* | 3,0 |
| 3* | 1,7 |
| 4* | 2,5 |
| 5* | 1,5 |

| | |
|---|---|
| * = erfindungsgemäß | |

Der Massenverlust von Folien mit den erfindungsgemäßen Weichmachern ist geringer als der von Folien, welche die Trimethylester enthalten.

### Beispiel 8 - Messung der Glasübergangstemperatur

Die Glasüberganstemperatur wurde mit Hilfe von DMTA-Messungen gemäß DIN 65583 mit einem Rheometer der Firma Anton Paar vom Typ MCR 302 bestimmt. Unter konstanten dynamischmechanischen Bedingungen (1 Hz, Deformation 0,3 %) wurden die viskoelastischen Eigenschaften der Folien (1 mm Dicke) in Abhängigkeit der Temperatur (Temperaturrampe von -100 bis +50 °C) erfasst und der Speichermodul, das Verlustmodul und der Verlustfaktor bestimmt. Das Maximum des Verlustmoduls wird dabei als Glasübergangstemperatur TG ausgewertet (s. Tabelle 7).

**Tabelle 7: Glasübergangstemperaturen der Folien**

| Foliennummer | Tg in °C |
|---|---|
| 1 | -18,6 |
| 2* | -25,2 |
| 3* | -30,5 |
| 4* | -27,3 |
| 5* | -37,4 |

| | |
|---|---|
| * = erfindungsgemäß | |

Je höher der C5- bzw. C9-Anteil der erfindungsgemäßen Folien ist, desto niedriger fallen die Glasübergangstemperaturen aus.

## Patentansprüche

1. Mischung von Triestern der 1,2,4-Cyclohexantripropionsäure gemäß der nachfolgenden Formel (1) 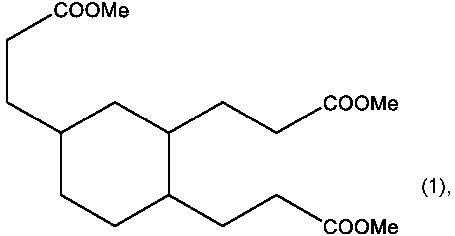 wobei in der Mischung 5 bis 75 Mol% der Reste R Methylreste und 25 bis 95 Mol% der Reste R ein C2- bis C10-Alkylrest sind.

2. Mischung nach Anspruch 1, wobei in der Mischung 15 bis 70 Mol%, vorzugsweise 20 bis 65 Mol% der Reste R Methylreste und 30 bis 85 Mol%, vorzugsweise 35 bis 80 Mol% der Reste R ein C2-bis C10-Alkylrest sind.

3. Mischung nach Anspruch 1 oder 2, wobei der C2- bis C10-Alkylrest ein C5- bis C9-Alkylrest, besonders bevorzugt ein C4- bis C9-Alkylrest, ganz besonders bevorzugt eine Pentylgruppe oder eine Isononylgruppe ist.

4. Weichmacherzusammensetzung, die eine Mischung von Triestern der 1,2,4-Cyclohexantripropionsäure gemäß der nachfolgenden Formel (1) 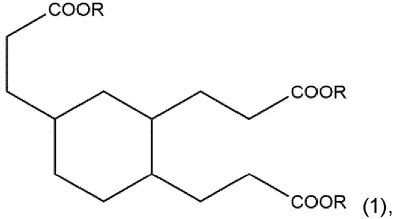 wobei in der Mischung 5 bis 75 Mol%, bevorzugt 15 bis 70 Mol%, besonders bevorzugt 20 bis 65 Mol% der Reste R Methylreste und 25 bis 95 Mol%, bevorzugt 30 bis 85 Mol%, besonders bevorzugt 35 bis 80 Mol% der Reste R ein C2- bis C10-Alkylrest, vorzugsweise ein C5- bis C9-Alkylrest, besonders bevorzugt ein C4- bis C9-Alkylrest, ganz besonders bevorzugt eine Pentylgruppe oder eine Isononylgruppe sind, und einen weiteren Weichmacher enthält.

5. Weichmacherzusammensetzung nach Anspruch 4, wobei die Mischung von Triestern der 1,2,4-Cyclohexantripropionsäure gemäß der Formel (1) und der weitere Weichmacher in einem Gewichtsverhältnis (Mischung von Triestern der 1,2,4-Cyclohexantripropionsäure gemäß der Formel (1) : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

6. Weichmacherzusammensetzung nach Anspruch 4 oder 5, wobei der weitere Weichmacher aus der Gruppe, bestehend aus Adipaten, Benzoaten, beispielsweise Monobenzoaten oder Glycoldibenzoaten, chlorierten Kohlenwasserstoffen, Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Cyclohexandicarboxylaten, Trimellitaten, Phthalaten und oligomeren oder polymeren Estern auf Basis von Adipin-, Bernstein- oder Sebacinsäure, ausgewählt wird.

7. Weichmacherzusammensetzung nach einem der Ansprüche 4 bis 6, wobei die Weichmacherzusammensetzung zusätzlich ein epoxidiertes Öl oder einen epoxidierten Alkylester einer Fettsäure enthält.

8. Weichmacherzusammensetzung nach Anspruch 7, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

9. Weichmacherzusammensetzung nach Anspruch 8, wobei das epoxidierte Öl epoxidiertes Sojabohnenöl oder epoxidiertes Leinöl, vorzugsweise epoxidiertes Sojabohnenöl ist.

10. Weichmacherzusammensetzung nach einem der Ansprüche 7 bis 9, wobei das epoxidierte Öl in einer Menge von 1 bis 150 Gewichtsteilen bezogen auf 100 Gewichtsteile der Gesamtsumme aus der Verbindung gemäß Formel (1) und dem Dialkylterephthalsäureester in der Weichmacherzusammensetzung vorhanden ist.

11. Kunststoffzusammensetzung, umfassend die Mischung nach einem der Ansprüche 1 bis 3 oder die Weichmacherzusammensetzung nach einem der Ansprüche 4 bis 10 und einen Kunststoff.

12. Kunststoffzusammensetzung nach Anspruch 11, wobei der Anteil der Mischung oder Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 13 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.
